(19) Europäisches Patentamt
European Patent Office
Office européen des brevets

(11) EP 0 793 476 B1

(12) # FASCICULE DE BREVET EUROPEEN

(45) Date de publication et mention de la délivrance du brevet:
**10.10.2001 Bulletin 2001/41**

(21) Numéro de dépôt: **95941138.0**

(22) Date de dépôt: **23.11.1995**

(51) Int Cl.[7]: **A61K 7/06**, A61K 7/42, A61K 31/70, A61K 35/78

(86) Numéro de dépôt international:
**PCT/FR95/01552**

(87) Numéro de publication internationale:
**WO 96/16632 (06.06.1996 Gazette 1996/26)**

(54) **Utilisation de la mangiférine ou ses dérivés pour une application cosmétique**

Verwendung von Mangiferin oder seinen Derivaten zur kosmetischen Anwendung

Use of Mangiferin or Derivatives thereof for Cosmetic Applications

(84) Etats contractants désignés:
**AT BE CH DE DK ES FR GB GR IE IT LI LU MC NL PT SE**

(30) Priorité: **25.11.1994 FR 9414176**
**24.03.1995 FR 9503513**
**22.06.1995 FR 9507508**

(43) Date de publication de la demande:
**10.09.1997 Bulletin 1997/37**

(73) Titulaire: **LABORATOIRES DE BIOLOGIE VEGETALE YVES ROCHER**
**56200 La Gacilly (FR)**

(72) Inventeurs:
- **ROUILLARD, Françoise**
**F-91160 Longjumeau (FR)**
- **JOSSE, Annabelle**
**F-94320 Thiais (FR)**
- **ROBIN, Jean-Renaud**
**F-93200 Saint-Denis (FR)**

(74) Mandataire: **Le Guen, Gérard et al**
**CABINET LAVOIX**
**2, place d'Estienne d'Orves**
**75441 Paris Cédex 09 (FR)**

(56) Documents cités:
**BE-A- 689 583**
**DE-A- 3 141 970**
**FR-A- 2 486 941**
**GB-A- 2 102 290**
**US-A- 4 217 341**

- **PHYTOTHER. RES., vol. 7, no. 2, 1993 pages 107-110, XP 000564924 S. GUHA ET AL. 'Activation of peritoneal macrophages by mangiferin, a naturally occuring xanthone.'**
- **CHEM. PHARM. BULL., vol. 40, no. 3, 1992 pages 721-724, XP 000564574 T. SATO ET AL 'Mechanism of antioxidant action of pueraria glycoside (PG)-1 (an isoflavonoid) and mangiferin (a xanthonoid).'**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 793 476 B1

Printed by Jouve, 75001 PARIS (FR)

## Description

La présente invention concerne l'utilisation pour une application cosmétique de la mangiférine (ou ses dérivés) naturelle ou obtenue par synthèse chimique, enzymatique, ou par voie biotechnologique, à titre de principe actif, ainsi que l'utilisation de compositions contenant à titre de principe actif un extrait végétal contenant de la mangiférine, en particulier un extrait de feuille d'Aphloïa ou de Mangiféra.

Ces compositions peuvent être utilisées notamment pour protéger la peau contre les rayonnements ultra-violets et le vieillissement cutané et améliorer sa qualité structurelle.

La mangiférine est un C-glucoside de la tétrahydroxy-1,3,6,7 xanthone. Elle est également nommée aphloïol (Billet and al, 1965). Cette molécule ainsi que ses dérivés (dérivés méthylés, O-glucosyles ou l'isomangiférine) sont naturellement présents dans un certain nombre de plantes,mais c'est la mangiférine qui est la C-glucoside xanthone la plus largement distribuée (Hostettmann, 1977), et plus particulièrement parmi les angiospermes.

Elle présente la structure chimique ci-dessous :

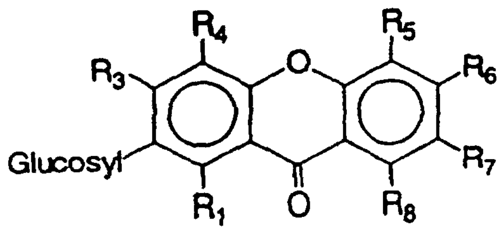

où $R_1 = R_3 = R_6 = R_7 = OH$ et $R_4 = R_5 = R_8 = H$.

On trouve également naturellement l'isomangiférine qui présente la structure ci-dessous :

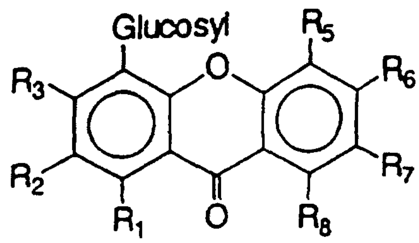

où $R_1 = R_3 = R_6 = R_7 = OH$ et $R_2 = R_5 = R_8 = H$.

Ses dérivés d'origine naturelle peuvent présenter des groupements O-méthyle ($-OCH_3$) ou glucosyle ($-C_6H_{11}O_6$) sur les positions $R_1$, $R_3$, $R_6$ ou $R_7$.

L'ensemble des composés formés par la mangiférine et ses dérivés) correspondent à la formule I générale suivante :

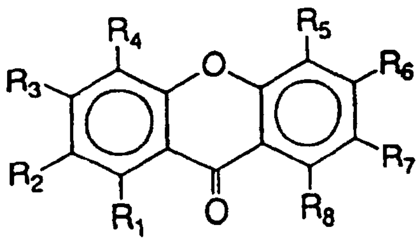

(I)

où $R_1$, $R_2$, $R_3$, $R_4$, $R_5$, $R_6$, $R_7$ sont choisis parmi -H, -OH, $-OCH_3$ et un radical glucosyle.

Les composés de formule I peuvent être obtenus par différents moyens :

1) extraction de matières végétales. La mangiférine et ses dérivés sont naturellement présents dans différentes

plantes, dont en particulier les espèces indiquées dans le tableau suivant :

| CLASSE | SOUS-CLASSE | ORDRE | FAMILLE | ESPECE |
|---|---|---|---|---|
| Filices | Leptosporangiatae | Filicales | Polypodiaceae | *Athyrium mesosorum* |
| Dicotyledoneae | Archichlamydeae | Guttiferales | Guttiferae | *Hypericum acutum* |
| | | | | *H. chinense* |
| | | | | *H. humifusum* |
| | | | | *H. montanum* |
| | | | | *H. nummularium* |
| | | | | *H. pulchrum* |
| | | Rosales | Leguminosae | *Hedysarum obscurum* |
| | | Rutales | Malphighiaceae | *Hiptage madablota* |
| | | Sapindales | Anacardiaceae | *Mangifera indica* |
| | | Celastrales | Hippocrateaceae | *Salacea prunoides* |
| | | Violales | Flacourtiaceae | *Flacourtia indica* |
| | | | | *Aphloïa theaeformis* |
| | | | | *A. madagascariensis* |
| | Sympetalae | Ebenales | Sapotaceae | *Madhuca utilis* |
| | | Tubiflorae | Convolvulacea | *Cuscuta reflexa* |
| Monocotyledoneae | | Liliiflorae | Liliceae | *Anemarrhena rhizoma* |
| | | | | *Smilax glycyphylla* |
| | | | Iridaceae | All spp. of *pogoniris* section |
| | | | | *Iris pseudacorus* |
| | | | | *Iris dichotoma* |
| | | | | *Belamcanda chinensis* |
| | | | | *Crocus aureus* |
| | | Graminales | Gramineae | ***Cymbopogon*** *afronardus* |

Les espèces d'Aphloïa et de Mangiféra sont des plantes dont les feuilles sont riches en mangiférine.

- L'Aphloïa est un arbuste entièrement glabre,de 3 à 4 m de hauteur, originaire de la côte est de Madagascar et des îles voisines (Réunion, Maurice, Seychelles, Comores). Les feuilles alternes sont simples, lancéolées, plus ou moins dentées sur les bords sauf à la base et non stipulées.

  Les fleurs hermaphrodites, solitaires, en cymes pauciflores, sont apétales. Elles possèdent 4 à 6 sépales imbriqués; les plus internes sont pétaloïdes.

  Les fruits sont des baies, blanches à maturité.

  Les feuilles de cette plante sont connues pour leurs propriétés diurétiques, veino-toniques et cicatrisantes qui les ont fait utiliser en infusion en médecine traditionnelle.
- Le Mangifera, et en particulier Mangifera indica, appartient à la famille des Anacardiacées. Le genre Mangifera est caractérisé par des caractères communs suivants : arbres à feuilles alternes, pétiolées, entières, coriaces aux inflorescences terminales en panicules. Les fruits sont des drupes.

  Ce genre comprend soixante deux espèces arborescentes dont 16 espèces donnent des fruits.

  L'espèce Mangifera indica L. en particulier est un arbre érigé à port plus ou moins étalé de 9 à 30 m de haut, toujours vert. Cette espèce comporte actuellement environ 1 000 variétés. Les espèces de Mangifera produisant des fruits sont très répandues. On trouve le Mangifera indica en particulier en Asie, Amérique Centrale et du Sud et en Afrique Tropicale.

  Traditionnellement, le fruit du Mangifera est consommé par les populations locales qui utilisent également d'autres parties de l'arbre pour divers usages : Le Mangifera indica en particulier est utilisé couramment pour soigner les populations : des infusions de l'écorce pour soigner la leucorrhée, les hémorragies et la dysenterie. Les feuilles sont employées en décoction pour la perte de voix, pour le diabète. Les cendres des feuilles sont également appliquées sur les brûlures.

  Les composés de formule I sont par exemple obtenus par purification d'extraits de tout ou partie de ces

plantes, selon tout procédé d'extraction et de purification (par exemple, extraction par un solvant polaire tel que l'eau, un alcanol, ou mélange de ces solvants, puis purification par cristallisation ou tout autre procédé connu de l'homme de l'art). Quelques-uns de ces procédés sont décrits par exemple dans le brevet publié sous le numéro FR-A-2 486 941.

2) par voie chimique ou enzymatique (procédés décrits entre autres dans deux articles : *Bhatia-V-K and al*, *Tetrahedron lett. (14)*, *p. 1741-2 et Nott-P-E, Phytochemistry*, *vol 6 (11); p. 1597-9).*
3) par toute voie biotechnologique : on peut envisager la culture de cellules de Mangiféra, en cals sur supports solides ou en fermenteurs, ou sous forme de protoplastes. Mais on peut également envisager d'utiliser la bioconversion de précurseurs de la mangiférine ou de ses dérivés par des microorganismes (par exemple des levures, bactéries...) et son obtention par extraction de ces microorganismes, ou par excrétion dans le milieu de culture.

La mangiférine (ou ses dérivés) peut être utilisée à différents degrés de pureté, seule ou en mélange.
La mangiférine purifiée sèche, se présente sous forme d'aiguilles prismatiques de couleur jaune. Elle ne possède ni odeur, ni saveur.
Elle est très peu soluble dans l'eau froide, soluble dans les liquides alcalins. Sa solubilisation est due à la formation de sels par les "OH" phénoliques et la mangiférine (ou ses dérivés) peut être employée sous forme libre ou de ses sels.
Les auteurs de la présente invention ont étudié les propriétés biologiques de la mangiférine (et ses dérivés) sous forme pure et sous forme d'extraits végétaux. En particulier, ceux-ci se sont intéressés aux activités d'extraits d'Aphloïa ou de Mangiféra dont les feuilles sont riches en mangiférine.
Les inventeurs ont à présent découvert que la mangiférine et ses dérivés sous forme purifiée ou contenus dans des extraits végétaux, en particulier les extraits de feuille d'Aphloïa ou de Mangiféra, possèdent des activités anti-ultra-violets (UV), anti-collagènase, anti-élastase très prononcées. Ces composés de formule I ont également des propriétés anti-radicalaire et anti-tyrosinase. Ils sont donc particulièrement utiles dans des compositions cosmétiques destinées à la protection de l'épiderme contre les rayons ultraviolets, pour améliorer la qualité structurelle de la peau et pour apporter une aide à la lutte contre le vieillissement cutané biologique et/ou actinique.
En conséquence, l'invention a pour objet l'utilisation d'un composé de la formule I générale suivante pour une application cosmétique :

formule I

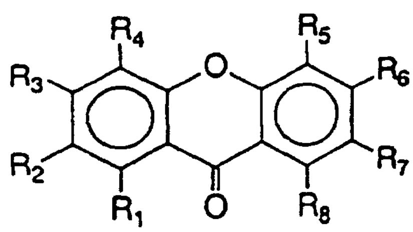

(I)

où $R_1$, $R_2$, $R_3$, $R_4$, $R_5$, $R_6$, $R_7$, $R_8$ sont choisis parmi -H, -OH, $-OCH_3$ et un radical glucosyle
ou un de ses sels pharmaceutiquement acceptable.
Selon une variante avantageuse, les compositions de la présente invention comprennent à titre de principe actif un composé de formule I,

où $R_1$, $R_3$, $R_6$, $R_7$, sont choisis parmi -H, -OH, $-OCH_3$ et un radical glucosyle,
et $R_2 = R_5 = R_8 =$ H et $R_4$ est un radical glucosyle,
ou $R_2$ est un radical glucosyle et $R_4=R_5=R_8=$-H.

Préférentiellement encore, $R_1=R_3=R_6=R_7=$OH, $R_2$ est un radical glucosyle et $R_4 = R_5 = R_8 =$-H.
Selon un mode de réalisation avantageux, les compositions comprennent un composé de formule I obtenu à partir d'extrait végétal.
Ces compositions comprennent préférentiellement, en fonction du composé choisi, des proportions allant de 0,01 à 50 % et préférentiellement encore de 0,01 à 10 % en poids de ce composé sous forme sèche.
L'invention a également pour objet l'utilisation de compositions cosmétiques comprenant un extrait de feuilles d'Aphloïa ou de Mangiféra en tant que principe actif, notamment des extraits obtenus par extraction avec des solvants

polaires.

Les compositions comprenant un extrait d'Aphloïa ou de Mangiféra, comprennent généralement de 0,1 à 20 % en poids d'extrait sec par rapport au poids total de la composition. La proportion d'extrait peut toutefois aller jusqu'à 50 % dans le cas d'un extrait liquide.

Les compositions selon l'invention ont un effet photoprotecteur au niveau de la peau, des lèvres ou des cheveux. Elles sont également destinées à améliorer la qualité structurelle de la peau, ou à lutter contre le vieillissement de la peau.

Les compositions selon l'invention se présentent de préférence sous forme d'émulsion simple Huile/Eau ou Eau/Huile, émulsions multiples ou micro-émulsions, gels aqueux, hydroalcooliques, huiles, lotions aqueuses ou hydroalcooliques, crème, bâtonnet, shampooing ou après-shampooing.

L'invention a également pour objet une méthode de traitement esthétique consistant à appliquer sur la peau, les cheveux ou les lèvres, une quelconque de ces compositions cosmétiques.

Elle a en outre pour objet l'utilisation d'un composé de formule I (mangiférine ou ses dérivés) ou d'un extrait de feuilles d'Aphloïa ou de Mangiféra pour la fabrication de compositions cosmétiques à action photoprotectrice, anti-collagénase et anti-élastase, anti-radicalaire et anti-tyrosinase.

On décrira ci-après les propriétés des extraits d'Aphloïa et de Mangiféra ainsi que celle de la mangiférine purifiée, ces propriétés étant illustrées par les figures 1 à 7b, dans lesquelles :

- la figure la représente le spectre d'absorption de la lumière UV en fonction de la longueur d'onde d'un extrait d'Aphloïa ;
- la figure 1b représente le spectre d'absorption de la lumière UV en fonction de la longueur d'onde d'un extrait de Mangifera ;
- la figure 1c représente le spectre d'absorption de la lumière UV en fonction de la longueur d'onde de la mangiférine ;
- la figure 2a représente la photostabilité en fonction du temps d'irradiation d'un extrait d'Aphloïa comparé à un filtre solaire commercialisé, Parsol MCX ;
- la figure 2b représente la photostabilité en fonction du temps d'un extrait de Mangifera, comparé à deux filtres solaires de synthèse commercialisés (extrait de Mangiféra, Univul T150, benzophénone 3) ;
- la figure 2c représente la photostabilité en fonction du temps de la mangiférine comparée à un filtre solaire commercialisé (Parsol MCX) ;
- la figure 3a représente les courbes d'activité antiradicalaire d'un extrait d'Aphloïa et d'un témoin sans inhibiteur en fonction du temps ;
- la figure 3b représente les courbes d'activité antiradicalaire d'un extrait de Mangifera (0,25 % ext. sec) et d'un témoin sans inhibiteur en fonction du temps ;
- la figure 3c représente les courbes d'activité antiradicalaire de la mangiférine (0,05 %) et d'un témoin sans inhibiteur en fonction du temps ;
- la figure 4a représente les courbes d'activité anti-élastase d'un extrait d'Aphloïa et d'un témoin sans inhibiteur (0,20 %, dil. 1/5) ;
- la figure 4b représente la courbe d'activité anti-élastase d'un extrait de Mangifera (0,25 %, dil. 1/4) déterminée par cinétique enzymatique au spectrophotomètre (mesure du rapport de l'absorbance sur le temps);
- la figure 4c représente la courbe d'activité anti-élastase de la mangiférine (0,01 %) ;
- la figure 5a représente l'activité anti-hyaluronidase d'un extrait d'Aphloïa ;
- la figure 5b représente l'activité anti-hyaluronidase d'un extrait de Mangifera (0,25 %) ;
- la figure 6 représente l'activité antityrosinase d'un extrait d'Aphloïa ;
- la figure 7a représente la courbe d'activité anti-collagénase d'un extrait de Mangifera (0,25 %) déterminée par la mesure de la densité optique à 320 nm;
- la figure 7b représente la courbe d'activité anti-collagènase de la mangiférine (0,042 %) ;

## I - EXTRAITS D'APHLOIA

L'extrait d'Aphloïa est obtenu à partir de toutes les sous-espèces d'Aphloïa, par exemple Aphloïa theaformis (Vahl) Benn, et Aphloïa madagascariensis Clos.

Les principaux constituants, actuellement identifiés dans l'extrait de feuilles d'Aphloïa de l'invention sont :

. un C-glucosyltétrahydroxyxanthane (l'Aphloïol)
. des flavonoïdes
. des tannins
. trois triterpène glucosides (ester glucosidique de l'acide tormentique et de l'acide 23 hydroxytormentique et ester glucosidique de l'acide 6-β-hydroxytormentique).

Il est exempt de substances alcaloïdes.

Les extraits d'Aphloïa peuvent être obtenus en soumettant les feuilles d'Aphloïa fraîches ou sèches, à une extraction par un solvant polaire, notamment par un solvant tel que l'eau, un alcanol (par exemple l'éthanol, le méthanol), le propylène glycol, le butylène glycol ou un mélange de ces solvants. On peut par exemple utiliser un mélange alcanol/eau ou bien un mélange propylène glycol/eau.

Le poids de solvant utilisé est de préférence égal à 2 à 20 fois le poids des feuilles rapporté au poids sec. On effectue avantageusement l'extraction, sous agitation, à une température située entre 10°C et la température d'ébullition du solvant. La durée de l'extraction est de préférence de 15 minutes à 5 heures.

On concentre éventuellement les solutions extractives, on sèche éventuellement les concentrats obtenus par les moyens connus de l'homme du métier (étuve à vide, micro-ondes...).

**Exemple d'extrait d'Aphloïa**

L'extrait étudié a été obtenu de la manière suivante :

6% (p/p) de feuilles sèches d'Aphloïa theaformis d'origine Malgache, grossièrement broyées, sont mis en contact sous agitation et à 55°C avec un mélange propylène glycol/eau (40/60). L'extraction se déroule pendant 1h30. La plante est écartée par filtration sur une toile (55 µm). Puis l'extrait est filtré sur un filtre plaque (5 µm).

Le pH de l'extrait se situe vers 5,5 et son extrait sec entre 0,8 et 1,3%.

**Effet photoprotecteur**

L'extrait d'Aphloïa décrit ci-dessus présente un spectre d'absorption dans l'UV très intéressant pour une application de protection contre l'érythème solaire, avec un maximum aux alentours de 320 nm, correspondant aux UVB et un autre aux environs de 360 nm, correspondant à la zone des UVA, comme représenté à la fig. 1a.

Par ailleurs, l'absence de phototoxicité a été vérifiée.

Compte tenu de l'innocuité de cet extrait, la résistance de l'extrait à l'irradiation, c'est à dire sa photostabilité, par rapport à celles de filtres synthétiques déjà commercialisés, comme par exemple l'octylméthoxycinnamate (PARSOL MCX, Givaudan, Bâle, Suisse) a été vérifiée.

Un filtre solaire est une molécule qui, par définition absorbe les radiations ultra violettes fortement énergétiques. Ainsi excitée, elle est susceptible de perdre son énergie, soit en émettant un rayonnement de longueur d'onde supérieur, soit en subissant des transformations intramoléculaires.

Ces réarrangements modifient ses propriétés physico-chimiques et en particulier sa faculté d'absorber la lumière dans la zone du spectre considéré.

L'extrait a donc été soumis, à un rayonnement continu dont le spectre est comparable à celui de la lumière solaire et de niveau énergétique contrôlable, émis par une lampe au xénon. L'évolution du spectre d'absorption a été suivie au cours du temps et le temps de demi-vie mesuré (durée d'irradiation nécessaire pour une perte d'absorption de 50% à une longueur d'onde fixée).

Comme représenté à la fig. 2a, l'extrait d'Aphloïa, comparé au PARSOL MCX dans les mêmes conditions s'est révélé aussi résistant à l'irradiation avec un temps de demi-vie très comparable.

Enfin l'extrait d'Aphloïa préparé selon le procédé précédemment décrit a été testé dans une composition cosmétique afin d'évaluer l'indice de protection SPF, selon la méthode SOLATEX, méthode d'évaluation du facteur de protection solaire in vitro commercialisée par IN VITRO INTERNATIONAL (Irvine, Californie, USA).

Une base cosmétique contenant 10% en poids de l'extrait ci-dessus a été comparée à la même base contenant 10% en poids de propylène glycol pur. Après application du protocole SOLATEX, les résultats suivants ont été obtenus :

base + propylène glycol ⇒ SPF = 1

base + extrait de l'invention ⇒ SPF = 4,5

L'extrait de l'invention s'avère donc efficace pour protéger la peau contre l'irradiation ultra violette; l'extrait peut être utilisé seul, ou en association avec des filtres chimiques synthétiques ou des écrans minéraux, dont il vient renforcer l'efficacité.

D'autre part les tests d'activité in vitro suivants montrent l'intérêt de l'extrait obtenu selon l'invention dans la protection de la peau au niveau moléculaire.

**Activité anti radicalaire** (déterminée selon V. Ponti, M.U. Dianzani, K. Cheeseman and T.F. Slater, Cehm - Biol interaction, 23 (1978) 281-291).

Des anions superoxydes sont générés par la réaction de la NADH (Nicotinamide Adénine Dinucléotide sous forme réduite) sur le PMS (Phénazine Méthosulfate). Les anions superoxydes réduisent le NBT (Nitro bleu de Tetrazolium) en diformazan de couleur bleu-violet.

L'apparition de NBT réduit est suivi dans le temps, au spectrophotomètre. Les résultats sont représentés sur la figure 3a.

On exprime l'activité anti-radicalaire des extraits en équivalent rutine pour une inhibition de 50%. Un extrait d'Aphloïa (extrait sec à 1%) présente une activité 6 à 12 fois plus importante que le témoin rutine (0,1 %) en fonction de la polarité du solvant d'extraction utilisé.

L'efficacité des extraits considérés dans la protection par rapport aux radicaux superoxides est tout à fait significative.

Compte tenu de la part active des radicaux libres dans le processus du vieillissement, l'extrait d'Aphloïa a potentiellement une activité intéressante dans la protection de la peau contre le vieillissement dû à l'altération des structures moléculaires de l'épiderme.

D'autre part, les extraits d'Aphloïa ont une activité protectrice par rapport aux protéines de structure : élastine et collagène.

**Détermination de l'activité anti-élastase** (déterminée selon J. Bieth, B. Spices et Camille G. Wermecth, Biochemical medecine 11, 350-357, 1974, modifié).

Une élastase pancréatique est mise à agir sur un substrat hydrophile, spécifique de l'enzyme MeO Suc - Ala - Ala - Pro - Val - pNa et ayant la caractéristique de libérer un produit chromogène, le p-nitroanilide (pNa). La cinétique enzymatique est suivie au spectrophotomètre. Les résultats sont représentés à la fig. 4a.

L'activité anti-élastase est exprimée en unité arbitraire et est définie par l'inverse de la quantité d'extrait pour obtenir une inhibition de 50 %, multiplié par 100.

Ainsi 200 µl de l'extrait ci-dessus dilué au 1/5ème permettent d'obtenir 50% d'inhibition par rapport au témoin sans inhibiteur. L'activité est donc de $\frac{1}{200}$ x 100 x 5 = 2,5 unités arbitraires.

L'extrait d'Aphloïa (1% d'extrait sec) présente une activité de 1 à 6 unités arbitraires.

**Activité anti-collagénase** (déterminée selon Erich Wünsels and Hans Georg Heidrich 15.07.1963).

Une collagénase de Clostridium histolycum est mise à agir sur un substrat hydrophile (p. phenylazo - benzylcarbonyl - L propyl - L leucyl - glycyl - L Propyl - D arginine). Le produit résultant de la coupure entre Leu et Gly est lipophile et absorbe la lumière UV à 320 nm.

L'activité anti-collagénase est exprimée en unités arbitraires et correspond à l'inverse de la quantité d'extrait (1% extrait sec) nécessaire pour obtenir 50% d'inhibition multipliée par 100. Pour l'extrait d'Aphloïa l'activité est de 0,5 à 3.

**Activité anti-hyaluronidase** (déterminée selon José L. Reissig, Jack L. Strominger and Luis F. Leloior, 05.12.1955).

La N-acétyl glucosamine libérée au bout de 45 minutes lors de la réaction de la hyaluronidase sur l'acide hyaluronique est mesurée au spectrophotomètre. Un procédé de réduction des sucres anhydre, qui en milieu acide sont transformés en dérivés furanne est utilisé; ces derniers réagissent avec le para-diméthyl- aminobenzaldéhyde pour former un complexe coloré.

L'activité est exprimée en µg de N-acétylglucosamine formée par minute.

L'activité anti hyaluronidase est exprimée en quantité d'extrait pour obtenir 50% d'inhibition.

Les résultats sont exprimés à la figure 5a.

Le volume de demi-inhibition $VI_{50}$, de l'extrait d'Aphloïa est de 95 à 120 µl. A titre de comparaison l'héparine, inhibiteur reconnu de la hyaluronidase (Sigma 10 USP/ml) a une $VI_{50}$ de 380 µl.

**Activité antityrosinase** (déterminée selon la méthode modifiée de Hamada, T et Mishima, Y(1972) British Journal of Dermatology, 86, 385).

Dans cette technique, la dopamine est mise en contact avec une tyrosinase en milieu tamponné à pH7 et transformée en dopachrome. Le dopachrome est une molécule colorée qui absorbe à 475 nm. La formation du produit est ainsi suivie dans le temps à 475 nm (voir figure 6 ).

L'activité de la tyrosinase est traduite par la vitesse de formation du produit. On détermine la concentration en extrait sec suffisante pour diminuer la vitesse de 50 %. (CI 50).

La CI 50 déterminée est de l'ordre de $5.10^{-2}$-0,3 mg/ml. Ce qui représente une activité 10 à 15 fois supérieure à celle obtenue avec un extrait de Busserole, déjà connu pour son activité antityrosinase.

Ces donnnées permettent de conclure que l'extrait d'Aphloïa possède une activité antityrosinase très intéressante. Par conséquent, en plus des utilisations déjà définies, les extraits sont donc utilisables en cosmétique de

soins et/ou de décoration et en pharmacie, pour aider à la résorption des taches brunes dues au vieillissement cutané et d'une manière plus générale au blanchiment cutané.

Les extraits d'Aphloïa possèdent un effet protecteur vis à vis des agressions moléculaires au niveau de la peau tout à fait considérable qui permet d'envisager leur application comme cosmétique en particulier pour la protection de la peau et des cheveux contre les rayons ultraviolets, pour lutter contre le vieillissement cutané dû à l'âge et de manière générale pour améliorer l'apparence et la qualité de la structure de l'épiderme.

**II - EXTRAITS DE MANGIFERA**

L'extrait de Mangiféra est obtenu à partir de toutes les espèces de Mangifera, et en particulier Mangifera indica L.

Les principaux constituants, actuellement identifiés dans l'extrait de feuilles de Mangifera sp. de l'invention sont :

. un C-glucosyltétrahydroxanthane
. des flavonoïdes
. des tannins

(Il est exempt de substances alcaloïdes).

L'extrait de Mangiféra est obtenu de la même façon que l'extrait d'Aphloïa, en soumettant les feuilles fraîches ou sèches à une extraction par un solvant polaire ou un mélange de solvants polaires (alcanol/eau ou propylène/eau par exemple). Ces extraits peuvent être utilisés sous forme liquide plus ou moins concentrés ou sous forme sèche.

**Exemple d'extrait de Mangifera**

L'extrait étudié a été obtenu de la manière suivante :

5% (p/p) de feuilles sèches de Mangifera indica, grossièrement broyées, sont mis en contact sous agitation et à 50°C avec un mélange éthanol/eau (30/70). L'extraction se déroule pendant 1h30. La plante est écartée par filtration sur une toile (55 µm). Puis l'extrait est filtré sur un filtre de cellulose (5 µm).

**Effet photoprotecteur**

L'extrait de Mangifera décrit ci-dessus présente un spectre d'absorption dans le domaine des UV très intéressant pour une application de protection contre l'érythème solaire, avec un maximum aux alentours de 320 nm, correspondant aux UVB et un autre aux environs de 360 nm, correspondant à la zone des UVA, comme représenté à la figure 1b.

Par ailleurs, l'absence de phototoxicité a été vérifiée.

Compte tenu de son innocuité, la résistance de l'extrait à l'irradiation (sa photostabilité) a été testée par rapport à celles des filtres synthétiques déjà commercialisés, (comme par exemple la benzophénone 3, GAF, USA ou l'UVINUL T150, BASF, Allemagne) dans des solvants compatibles à leurs solubilités respectives.

L'extrait a donc été soumis à un rayonnement continu dont le spectre est comparable à celui de la lumière solaire et de niveau énergétique contrôlable, émis par une lampe au xénon. L'évolution du spectre d'absorption a été suivie au cours du temps et le temps de demi-vie mesuré (durée d'irradiation nécessaire pour une perte d'absorption de 50% à une longueur d'onde fixée).

Comme représenté à la figure 2b, l'extrait de Mangifera, comparé à la benzophénone 3, et à l'UVINUL T150 dans les mêmes conditions, s'est révélé au moins aussi résistant à l'irradiation, avec un temps de demi-vie nettement supérieur à ces deux filtres synthétiques.

Enfin, l'extrait de Mangifera préparé comme décrit dans le procédé de l'invention a été testé dans une composition cosmétique afin d'évaluer l'indice de protection SPF, selon la méthode SOLATEX, méthode d'évaluation du facteur de protection solaire in vitro commercialisée par IN VITRO INTERNATIONAL (Irvine, Californie, USA).

On a incorporé 5 % en poids de l'extrait ci-dessus à une base cosmétique (contenant en outre 1.5 % de Parsol 1789, 4 % de Parsol MCX, 2 % d'Eusolex 507 et 2 % d'oxyde de titane Tioveil fin, lui conférant un SPF initial égal à 8). Cette préparation a été comparée à la même base contenant 5% en poids de propylène glycol pur. Après application du protocole SOLATEX, les résultats suivants ont été obtenus :

base seule ⇒ SPF = 8
base + extrait de l'invention ⇒ SPF = 14

L'extrait de l'invention s'avère donc efficace pour protéger la peau contre l'irradiation ultra violette; l'extrait peut être utilisé seul, ou en association avec des filtres chimiques synthétiques ou des écrans minéraux, dont il vient

renforcer l'efficacité.

L'intérêt de l'extrait obtenu selon l'invention pour la protection de la peau au niveau moléculaire est également illustré par les exemples suivants :

**Activité anti radicalaire déterminée in vitro** (V. Ponti, M.U. Dianzani, K. Cheeseman and T.F. Slater, Chem. Biol. interaction, 23, 281-291, 1978).

La technique utilisée est la même que celle décrite pour l'extrait d'Aphloïa. Les résultats sont représentés sur la figure 3b.

On exprime l'activité anti-radicalaire des extraits en équivalent rutine pour une inhibition de 50%. Un extrait de Mangifera (extrait sec 1%) présente une activité 15 à 30 fois plus importante que le témoin rutine (0,1 %) en fonction de la polarité du solvant d'extraction utilisé.

Les données obtenues montrent que l'efficacité des extraits considérés dans la protection par- rapport aux radicaux superoxydes est tout à fait significative.

Les radicaux libres étant connus pour leur part active non négligeable dans le processus de vieillissement, l'extrait selon l'invention a donc potentiellement une activité intéressante dans la protection de la peau contre le vieillissement dû à l'altération des structures moléculaires de l'épiderme.

**Activité anti-élastase déterminée in vitro** (J. Bieth, B. Spices et Camille G. Wermecth, Biochemical medecine 11, 350-357, 1974) Modifié.

Le protocole suivi est le même que celui décrit pour l'extrait d'Aphloïa.

La courbe des pentes obtenue en fonction des quantités d'extrait de Mangiféra, permet ainsi de définir la concentration correspondant à 50 % d'inhibition (CI50). Cette courbe est reportée sur la figure 4b.

L'extrait de Mangifera selon l'invention présente une CI50 très significative, de l'ordre de $5.10^{-2}$ - 1 mg/ml et pouvant aller au-delà de cet écart en fonction de l'espèce considérée.

**Activité anti-collagénase déterminée in vitro**( Erich Wünsch and Hans Georg Heidrich Hoppe-Seyler's - Zeit - Physiol. - Chem. 333, 149-151, 1963).

Une collagénase est mise à agir sur un substrat hydrophile (p. phenylazo - benzylcarbonyl - L propyl - L leucyl - glycyl - L Propyl - D arginine). Le produit résultant de la coupure entre Leu et Gly est lipophyle et absorbe la lumière UV à 320 nm.

L'effet inhibiteur de Mangifera est mesuré en ajoutant l'extrait obtenu selon l'invention au milieu réactionnel.

La figure 7a représente la courbe des valeurs obtenues à 320 nm en fonction des différents volumes d'extrait de Mangifera ajoutés au milieu.

La concentration permettant 50 % d'inhibition (CI50) peut être ainsi déterminée.

L'extrait de Mangiféra présente une CI50 de l'ordre de $5. 10^{-2}$ - 1 mg/ml et pouvant aller au-delà de cet écart en fonction de l'espèce considérée.

**Activité anti-hyaluronidase déterminée in vitro** (José L. Reissig, Jack L. Strominger and Luis F. Leloir, J. Biol.-Chem. 217,960-966, 1953).

Le protocole est le même que celui décrit pour l'extrait d'Aphloïa.

L'activité est exprimée en µg de N-acétylglucosamine formé par minute.

L'activité anti hyaluronidase est exprimée en quantité d'extrait pour obtenir 50% d'inhibition. Les résultats sont reportés sur la figure 5b.

Le volume de demi-inhibition de l'extrait de mangiféra (extrait sec 1 %) $VI_{50}$, est de 200-250 µl, en comparaison avec l'héparine, inhibiteur reconnu de la hyaluronidase (Sigma 10 USP/ml) dont $VI_{50}$ = 380 µl.

**Activité antityrosinase** (déterminée selon la méthode modifiée de Hamada, T et Mishima, Y (1972) British Journal of Dermatology, 86, 385).

Le protocole suivi est le même que celui décrit pour l'extrait d'Aphloïa. L'activité de la tyrosinase est traduite, par la vitesse du produit formé, mesuré à 475 mm.

On détermine la concentration en extrait sec suffisante pour diminuer la vitesse de 50 % : $CI_{50}$ de l'ordre de 2 à 3 mg/ml.

Ces données permettent de conclure que les extraits de Mangifera possèdent un effet protecteur vis à vis

des agressions moléculaires au niveau de la peau tout à fait considérable et par conséquent, l'utilisation de ceux-ci en cosmétique présente un intérêt évident, en particulier pour la protection de la peau et des cheveux contre les rayons ultra-violets, pour lutter contre le vieillissement cutané dû à l'âge et de manière générale pour améliorer l'apparence et la qualité de la structure de l'épiderme.

## III - MANGIFERINE PURIFIEE

### Exemple de préparation d'une solution de mangiférine à partir d'un extrait végétal

Un des procédés d'extraction de la mangiférine consiste à extraire avec de l'éthanol 40 % les glucosides de la xanthone de la plante broyée du genre Mangiféra indica. On effectue l'extraction à chaud (par exemple 50°C), à raison de 1 kg de matière végétale, pour 10 l d'agent d'extraction. L'extrait est partiellement évaporé et refroidi. La mangiférine cristallise et se dépose. On récupère ce dépôt que l'on rince plusieurs fois avec un mélange de solvants, tel l'eau ou le chloroforme, jusqu'à obtention de la substance purifiée.

### Effet photoprotecteur

La mangiférine (ou ses dérivés) présente un spectre d'absorption dans le domaine des UV très intéressant pour une application de protection contre l'érythème solaire, avec un maximum aux alentours de 320 nm, correspondant aux UVB et un autre aux environs de 360 nm, correspondant à la zone des UVA comme représenté à la figure 1c.
Par ailleurs, l'absence de phototoxicité a été vérifiée.
Compte tenu de son innocuité, la résistance de l'extrait à l'irradiation (sa photostabilité) a été testée par rapport à celles des filtres synthétiques déjà commercialisés (comme par exemple l'octylméthoxycinnamate, Parsol MCX, Givaudan, Bâle, Suisse).
Une solution de mangiférine a été soumise à un rayonnement continu dont le spectre est comparable à celui de la lumière solaire et de niveau énergétique contrôlable, émis par une lampe au xénon. L'évolution du spectre d'absorption a été suivie au cours du temps et le temps de demi-vie mesuré (durée d'irradiation nécessaire pour une perte d'absorption de 50 % à une longueur d'onde fixée).
Comme représenté à la figure 2c, la mangiférine en solution aqueuse comparée à la Benzophénone 4 dans les mêmes conditions, s'est révélée aussi résistante à l'irradiation avec un temps de demi-vie très comparable.
Qui plus est, son coefficient d'absorption moléculaire calculé se révèle comparable à ceux de la plupart des filtres synthétiques commercialisés, c'est-à-dire entre 20 000 et 30 000 $L.cm^{-1}.mole^{-1}$.
La mangiférine peut donc s'avérer efficace pour protéger la peau contre l'irradiation ultra-violette; elle peut être utilisée seule, ou en association avec des filtres chimiques synthétiques ou des écrans minéraux, dont elle vient renforcer l'efficacité.
L'intérêt de la mangiférine pour la protection de la peau au niveau moléculaire est également illustré par les exemples suivants :

**Activité anti-élastase déterminée in vitro** (*J. Bieth, B. spices et Camille G. Wermecht, Biochemical médecine 11, 350-357, 1974)* modifié.

Selon le même protocole que celui précédemment décrit pour les extraits d'Aphloïa et de Mangiféra, des quantités croissantes d'une solution de mangiférine (0,01%) sont introduites dans un mélange réactionnel final de 3,01 ml.
La courbe des pentes obtenue en fonction des quantités de mangiférine, permet ainsi de définir la concentration correspondant à 50 % d'inhibition (CI50). Cette courbe est reportée sur la figure 4c.
La mangiférine présente une CI50 très significative de l'ordre de $10^{-2}$ mg/ml.

**Activité anti-collagènase déterminée in vitro** (*Erich Wünsch and Hans Georg Heidrich Hoppe-Seyler's - Zeit - Physiol. Chem. 333*, *149-151*, *1963).*

L'effet inhibiteur de la mangiférine est mesuré par ajout au milieu réactionnel d'une solution à 0,042 % dans du propylène glycol.
La figure 7b représente la courbe des valeurs obtenues à 320 nm en fonction des différents volumes de solution de mangiférine ajoutés au milieu. La concentration permettant 50 % d'inhibition (CI50) peut être ainsi déterminée. La mangiférine présente un CI50 de l'ordre de $5.10^{-2}$ mg/ml.
Les données obtenues montrent que l'efficacité de cette molécule en particulier au niveau de la protection des qualités structurelles de la peau est tout à fait significative.

**Activité anti-tyrosinase in vitro**

Une tyrosinase est mise à agir à température ambiante, sur un substrat LDopa. Le produit formé est suivi au cours du temps au spectrophotomètre à 475 nm. Une solution de mangiférine à 0,2 % permet une inhibition de 50 % de la tyrosinase, soit une $CI_{50}$ de l'ordre de 0,2 mg/ml.

Par son effet anti-tyrosinase, la mangiférine peut contribuer à l'unification de l'aspect de la peau.

**Activité anti-radicalaire déterminée in vitro** (*V. Ponti, M.U. Dianzani*, *K. Cheeseman and T.F. Slater, Chem. Biol. interaction*, *23*, *281-291*, *1978).*

Les résultats sont représentés sur la figure 3c.

On exprime l'activité anti-radicalaire des extraits en équivalent rutine pour une nhibition de 50%.

Une solution de mangiférine à 1 % dans du propylène glycol présente une activité 4 fois plus importante qu'une solution référence de rutine à 0,1 %. Les données obtenues montrent que l'efficacité de cette molécule dans la protection par rapport aux radicaux superoxydes est intéressante.

L'ensemble de ces données permet de conclure que la molécule de mangiférine ou ses dérivés ainsi que les extraits d'Aphloïa ou de Mangiféra en contenant, sont utilisables en cosmétique de soins et/ou de décoration. Ils sont en particulier utiles : pour la protection contre les UVA et UVB, de par leur rôle filtrant et anti radicalaire, protégeant de l'effet nocif des radicaux produits sous l'effet des UVA; pour les peaux fragilisées par le temps (âge) : diminution de l'hydratation et de la tonicité des tissus, de par leurs propriétés anti-hyaluronidase limitant la dégradation de l'acide hyaluronique (protéoglycane ayant un pouvoir hydratant très puissant) ; ainsi que par leurs propriétés anti-collagénase et anti-élastase limitant la dégradation du collagène et de l'élastine, l'état de ces molécules étant notamment responsable de la qualité structurelle de la peau.

Les extraits de feuilles d'Aphloïa et de Mangifera sp. ainsi que la mangiférine sous forme pure et ses dérivés peuvent être utilisés tels quels, vectorisés, microencapsulés, en association avec un mélange d'excipients tels que : huiles végétales, minérales ; cires végétales ou minérales ; silicones ; alcools et acides gras ; agents tensio actifs ; dérivés de protéines, gélifiants inorganiques ou organiques ; lanoline et ses dérivés ; filtres UV organiques ou inorganiques, eau; ou en association avec d'autres extraits végétaux.

Les exemples suivants de composition contenant un extrait d'aphloïa, de Mangiféra ou de la Mangiférine purifiée pour principe actif, illustrent la présente invention.

**1. Exemples de compositions sous forme de gels**

Les tableaux suivants indiquent des formulations pour des compositions sous forme de gel aqueux (colonne 1) ou de gel hydroalcoolique (colonne 2),

- contenant de la Mangiférine purifiée :

| | (1) | (2) |
|---|---|---|
| Acide polyacrylique | 1,20 | 1,20 |
| Gomme Xanthane | 0,30 | 0,30 |
| Mangiférine | 0,01 à 10 % | 0,01 à 10 % |
| Eau | qsp | qsp |
| Ethanol | x | 50 |
| Triglycérides C8/C10 | 5 | x |
| Lanoline 78 OE * | x | 5 |
| Alcool olëique 200 E | 3 | x |
| Parfum | 0,20 | 0,20 |
| Triéthanolamine (TEA) | 1,10 | 1,10 |

* Lanoline éthoxylée avec 78 moles d'OE

- contenant un extrait de Mangiféra :

| | (1) | (2) |
|---|---|---|
| Acide polyacrylique | 1,20 | 1,20 |
| Hydroxypropyl | 0,20 | 0,20 |
| Extrait sec de Mangiféra | 0,01 à 10 % | 0,01 à 10 % |
| Eau | qsp | qsp |
| Ethanol | x | 40 |
| Acide Caprique 7 0E** | 3 | x |
| Diméthicone copolyol | x | 5 |
| Alcool laurique 14 0E** | 3 | x |
| Parfum | 0,20 | 0,20 |
| NaOH | 0,30 | 0,35 |

** Acide caprique éthoxylé avec 7 moles d'OE
*** Alcool laurique éthoxylé avec 14 moles d'OE

**2. Exemples de compositions sous forme d'émulsion**

- contenant de la Mangiférine purifiée :

| | | |
|---|---|---|
| A | Eau | qsp |
| | Mangiférine | 0,01 à10% |
| | Conservateurs | qsp |
| | Propylène glycol | 5,00% |
| | Gomme xanthane | 0,30% |
| | Copolymère acrylique/acrylate | 0,50% |
| B | Acide stéarique 100 OE **** | 3,00% |
| | Stéarate de sorbitan | 2,00% |
| | Sorbitan laurate 20 OE | 3,00% |
| | Alcool cétylstéarique | 1,50% |
| | Cire d'abeille | 1,00% |
| | Huile de germe de blé | 5,00% |
| | Diméthicone | 2,00% |
| | Cyclométhicone | 5,00 % |
| C | Gel de polyacrylamide | 2,00% |
| D | Parfum | 0,30% |

**** acide stéarique éthoxylé avec 100 moles d'OE

- contenant un extrait d'Aphloïa :

| | | |
|---|---|---|
| A | Eau | qsp |
| | Extrait sec d'Aphloïa | 0,01 à 10% |
| | Conservateurs | qsp |
| | Dipropylène glycol | 5,00% |
| | Alginate de sodium | 0,30% |
| | Copolymère acrylique/acrylate | 0,50% |
| | Acide stéarique 100 OE | 3,00% |
| | Stéarate de sorbitan | 2,00% |

(suite)

| | | |
|---|---|---|
| B | Sorbitan laurate 20 OE | 3,00% |
| | Acide stéarique | 1,50% |
| | Cire de Carnauba | 1,00% |
| | Huile d'aveline | 5,00% |
| | Diméthicone | 2,00% |
| | Cyclométhicone | 5,00% |
| C | Gel de polyacrylamide | 2,00% |
| D | Parfum | 0,30% |

**3. Exemples de compositions sous forme de crème**

- contenant de la Mangiférine purifiée:

| | | |
|---|---|---|
| A | Eau | qsp |
| | Gomme xanthane | 0,30% |
| | Séquestrant (par ex. EDTA) | 0,05% |
| | Conservateurs | qsp |
| | Mangiférine | 0,01 à10% |
| B | Acide C 18 | 2,50% |
| | Alcool C 16 | 2,50% |
| | Trilaurine | 1,00% |
| | Beurre de karité | 3,00% |
| | Acétate de tocophérol | 0,05% |
| | αBisabolol | 0,05% |
| | Huile végétale (blé) | 5,00% |
| | Diméthicone | 3,00% |
| | Acide polyacrylique | 0,30% |
| C | Eau | 3,00% |
| D | TEA | 1,50% |
| E | Parfum | 0,10% |

- contenant un extrait de Mangiféra :

| | | |
|---|---|---|
| A | Eau | qsp |
| | Gomme guar | 0,30% |
| | Séquestrant (par ex. EDTA) | 0,05% |
| | Conservateurs | qsp |
| | Extrait sec de Manguier | 0,01 à10% |
| B | Acide C 18 | 2,50% |
| | Céthyl palnitate | 2,50% |
| | Trilaurine | 1,00% |
| | Beurre de karité | 3,00% |
| | Acétate de tocophérol | 0,05% |
| | αBisabolol | 0,05% |
| | Cocoate d'éthyl hexyl | 5,00% |
| | Diméthicone | 3,00% |
| | Acide polyacrylique | 0,30% |
| C | Eau | 3,00% |

(suite)

| | | |
|---|---|---|
| D | TEA | 1,50% |
| E | Parfum | 0,10% |

**4. Exemples de compositions sous forme de lotion**

- contenant de la Mangiférine purifiée :

| | |
|---|---|
| Eau | qsp |
| Agent séquestrant | 0,05% |
| Propylène glycol | 2,00% |
| Conservateurs | qsp |
| Mangiférine | 0,01 à 10% |
| Alcool | 5 à 50% |
| Alcool olëique 20 OE | 1,00% |
| Parfum | 0,05% |
| Colorants | qsp |

- contenant un extrait d'Aphloïa :

| | |
|---|---|
| Eau | qsp |
| Agent séquestrant | 0,05% |
| Méthyl gluceth 20 (émollient) | 2,00% |
| Conservateurs | qsp |
| Extrait d'Aphloïa | 0,01 à 50% |
| Alcool | 5 à 50% |
| Huile de ricin 40 OE | 1,00% |
| Parfum | 0,05% |
| Colorants | qsp |
| Alanthoine | 0,1% |

**Revendications**

**1.** Utilisation d'un composé de la formule générale I suivante :

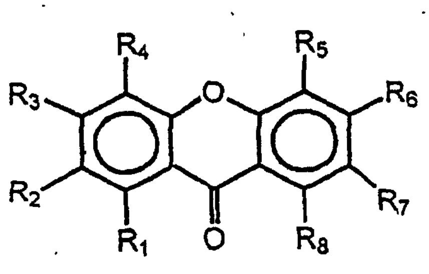

formule I

où $R_1$, $R_2$, $R_3$, $R_4$, $R_5$, $R_6$, $R_7$, $R_8$ sont choisis parmi -H, -OH, $-OCH_3$ et un radical glucosyle, ou d'un de ses sels pharmaceutiquement acceptable, pour une application cosmétique.

**2.** Utilisation d'un composé de formule I selon la revendication 1, dans laquelle $R_1$, $R_3$, $R_6$, $R_7$ sont choisis parmi -H, -OH, $-OCH_3$ et un radical glucosyle,

et $R_2 = R_5 = R_8 = H$ et $R_4$ est un radical glucosyle, ou $R_2$ est un radical glucosyle et $R_4= R_5= R_8= H$.

3. Utilisation d'un composé de formule I selon la revendication 1 ou 2, dans laquelle $R_1= R_3 = R_6 = R_7 = OH$ et $R_2$ est un radical glucosyle et $R_4= R_5= R_8= H$.

4. Utilisation d'un composé de formule I selon l'une quelconque des revendications 1 à 3, **caractérisée en ce que** ledit composé est mis en oeuvre au sein d'une composition cosmétique en une quantité allant de 0,01 à 50 % en poids, sous forme sèche.

5. Utilisation d'un composé de formule I selon la revendication précédente, **caractérisée en ce que** ledit composé est présent en une quantité allant de 0,01 à 10 % en poids, sous forme sèche.

6. Utilisation d'un composé de formule I selon l'une quelconque des revendications précédentes, **caractérisée en ce que** ledit composé est mis en ceuvre sous forme d'un extrait végétal.

7. Utilisation d'un composé de formule I selon la revendication 6, **caractérisée en ce que** ledit composé est mis en oeuvre sous forme d'un extrait de feuilles d'Aphloïa ou d'un extrait de feuilles de Mangiféra.

8. Utilisation d'un composé de formule I selon la revendication 7 **caractérisée en ce que** l'extrait de feuilles est mis en oeuvre dans une composition cosmétique à raison de 0,01 % à 20 % en poids, exprimé en poids d'extrait sec par rapport au poids total de la composition.

9. Utilisation d'un composé de formule I selon la revendication 6 ou la revendication 7, **caractérisée en ce que** l'extrait végétal est obtenu par extraction par un solvant polaire ou par un mélange de solvants polaires.

10. Utilisation d'un composé de formule I selon l'une quelconque des revendications précédentes, **caractérisé en ce que** ledit composé est mis en oeuvre dans une composition cosmétique se présentant sous forme d'une émulsion simple ou multiple, d'une micro-émulsion, d'un gel aqueux ou hydroalcoolique, d'une crème, d'une huile, d'une lotion aqueuse ou hydroalcoolique, d'un bâtonnet, d'un shampooing ou d'un après-shampooing.

11. Utilisation d'un composé de formule I selon l'une quelconque des revendications 1 à 10 pour protéger la peau, les lèvres ou les cheveux contre les rayons ultraviolets , pour améliorer la qualité structurelle de la peau ou pour lutter contre le vieillissement cutané.

12. Méthode de traitement esthétique consistant à appliquer sur la peau, les lèvres ou les cheveux, une composition cosmétique comprenant un composé de formule I tel que définie dans la revendication 1.

13. Utilisation d'un extrait de feuille d'Aphloïa obtenu par extraction par un solvant polaire ou un mélange de solvants polaires, ou d'un extrait de feuille de Mangiféra obtenu par extraction par un solvant polaire ou un mélange de solvants polaires, pour fournir un effet photoprotecteur, une action anti-radicalaire, une action anti-élastase, une action anti-collagénase, une action anti-hyaluronidase, ou une action anti-tyrosinase.

14. Utilisation de mangiférine purifiée, pour fournir un effet photoprotecteur, une action anti-radicalaire, une action anti-élastase, une action anti-collagénase, ou une action anti-tyrosinase.

**Patentansprüche**

1. Verwendung einer Verbindung der folgenden allgemeinen Formel I

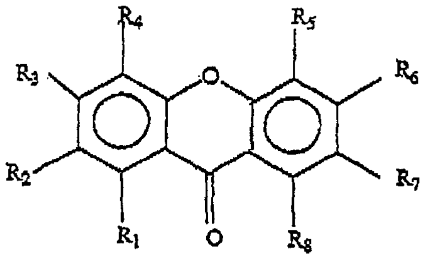

Formel I

worin $R_1$, $R_2$, $R_3$, $R_4$, $R_5$, $R_6$, $R_7$, $R_8$ ausgewählt sind aus -H, -OH, $-OCH_3$ und einem Glucosylrest oder einem seiner pharmazeutisch verträglichen Salze für eine kosmetische Zubereitung.

**2.** Verwendung einer Verbindung der Formel I gemäß Anspruch 1, worin $R_1$, $R_3$, $R_6$, $R_7$ ausgewählt sind aus -H, -OH, $-OCH_3$ und einem Glucosylrest und worin $R_2 = R_5 = R_8 =$ H und $R_4$ ein Glucosylrest ist oder $R_2$ ein Glucosylrest und $R_4 = R_5 = R_8 =$ H ist.

**3.** Verwendung einer Verbindung der Formel I gemäß Anspruch 1 oder 2, worin $R_1 = R_3 =$ R6 $= R_7 =$ OH ist und $R_2$ ein Glucosylrest und $R_4 = R_5 = R_8 =$ H ist.

**4.** Verwendung einer Verbindung der Formel I gemäß einem der Ansprüche 1 bis 3, **dadurch gekennzeichnet, daß** diese Verbindung in einer kosmetischen Zubereitung in einer Menge von 0,01 bis 50 Gew.% in trockener Form eingesetzt ist.

**5.** Verwendung einer Verbindung der Formel I nach den vorangegangenen Ansprüchen, **dadurch gekennzeichnet, daß** diese Verbindung in einer Menge von 0,01 bis 10 Gew.% in trockener Form enthalten ist.

**6.** Verwendung einer Verbindung der Formel I nach einem der vorangegangenen Ansprüche, **dadurch gekennzeichnet, daß** die Verbindung in Form eines tierischen Extrakts eingesetzt ist.

**7.** Verwendung einer Verbindung der Formel I nach Anspruch 6, **dadurch gekennzeichnet, daß** diese Verbindung in Form eines Extrakts aus Blättern der Aphloia oder eines Extrakts von Blättern von Mangifera eingesetzt ist.

**8.** Verwendung einer Verbindung der Formel I nach Anspruch 7, **dadurch gekennzeichnet, daß** der Blattextrakt in einer kosmetischen Zubereitung eingesetzt ist in einer Menge von 0,01 bis 20 Gew.%, ausgedrückt als Trockengewicht des Extrakts, bezogen auf das Gesamtgewicht der Zusammensetzung.

**9.** Verwendung einer Verbindung der Formel I nach Anspruch 6 oder Anspruch 7, **dadurch gekennzeichnet, daß** der pflanzliche Extrakt erhältlich ist durch Extraktion mit einem polaren Lösungsmittel oder einem Gemisch polarer Lösungsmittel.

**10.** , Verwendung einer Verbindung der Formel I nach einem der vorangegangenen Ansprüche, **dadurch gekennzeichnet, daß** diese Verbindung eingesetzt ist in einer kosmetischen Zubereitung, die in Form einer einfachen oder mehrfachen Emulsion, einer Mikroemulsion, einem wässrigen oder hydroalkoholischen Gel, einer Creme, einem Öl, einer wässrigen oder hydroalkoholischen Lotion, einem Stab, einem Shampo oder einem Aprés-Shampo vorliegt.

**11.** Verwendung einer Verbindung der Formel I nach einem der vorangegangenen Ansprüche 1 bis 10 zum Schutz der Haut, der Lippen oder der Haare gegen ultraviolette Strahlen, zur Verbesserung der Hautstruktur oder zur Bekämpfung der Alterung der Cuta.

**12.** Verfahren zur kosmetischen Behandlung, bestehend aus dem Aufbringen einer kosmetischen Zubereitung, die eine Verbindung der Formel I, definiert in Anspruch 1, enthält, auf die Haut, die Lippen oder die Haare.

**13.** Verwendung eines Extrakts des Blatts von Aphloia, erhalten durch Extraktion mit einem polaren Lösungsmittel oder einem Gemisch polarer Lösungsmittel, oder eines Extrakts des Blatts von Magnifera, erhalten durch Extraktion mit einem polaren Lösungsmittel oder einem Gemisch polarer Lösungsmittel, zur Bereitstellung einer Lichtschutzwirkung, einer Wirkung gegen Radikale, einer Antielastase-Wirkung, einer Anticollagenase-Wirkung, einer Antihyaluronidase-Wirkung oder einer Antityrosinase-Wirkung,

**14.** Verwendung von gereinigtem Magniferin zur Bereitstellung einer Lichtschutzwirkung, einer gegen Radikale gerichteten Wirkung, einer Antielastase-Wirkung, einer Anticollagenase-Wirkung oder Antityrosinase-Wirkung.

**Claims**

**1.** Use of a compound of the following general formula I:

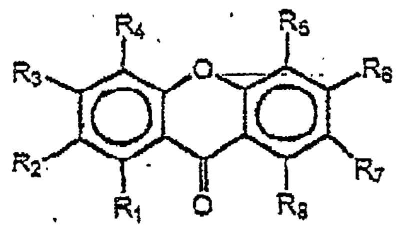

formula I

wherein $R_1$, $R_2$, $R_3$, $R_4$, $R_5$, $R_6$, $R_7$, $R_8$ are selected from -H, -OH, $-OCH_3$ and a glucosyl radical,
or of one of its pharmaceutically acceptable salts,
for a cosmetic application.

**2.** Use of a compound of formula I according to claim 1, wherein $R_1$, $R_3$, $R_6$, $R_7$ are selected from -H, -OH, $-OCH_3$ and a glucosyl radical,
and $R_2 = R_5 = R_8 = H$ and $R_4$ is a glucosyl radical, or $R_2$ is a glucosyl radical and $R_4 = R_5 = R_8 = H$.

**3.** Use of a compound of formula I according to claim 1 or 2, wherein $R_1 = R_3 = R_6 = R_7 = OH$
and $R_2$ is a glucosyl radical and $R_4 = R_5 = R_8 = H$.

**4.** Use of a compound of formula I according to any one of claims 1 to 3, **characterised in that** the compound is used in a cosmetic composition in an amount of from 0,01 to 50% by weight, in dry form.

**5.** Use of a compound of formula I according to the preceding claim, **characterised in that** the compound is present in an amount of from 0.01 to 10% by weight, in dry form.

**6.** Use of a compound of formula I according to any one of the preceding claims, **characterised in that** the compound is used in the form of a plant extract.

**7.** Use of a compound of formula I according to claim 6, **characterised in that** the compound is used in the form of an Aphloia leaf extract or a Mangifera leaf extract.

**8.** Use of a compound of formula I according to claim 7, **characterised in that** the leaf extract is used in a cosmetic composition in a proportion of from 0.01% to 20% by weight, expressed by weight of dry extract relative to the total weight of the composition.

**9.** Use of a compound of formula I according to claim 6 or claim 7, **characterised in that** the plant extract is obtained by extraction by a polar solvent or by a mixture of polar solvents.

**10.** Use of a compound of formula I accordinq to any one of the preceding claims, **characterised in that** the compound is used in a cosmetic composition which is in the form of a single or multiple emulsion, a micro-emulsion, an aqueous or aqueous-alcoholic gel, a cream, an oil, an aqueous or aqueous-alcoholic lotion, a stick, a shampoo or a conditioner.

**11.** Use of a compound of formula I according to any one of claims 1 to 10 for protecting the skin, the lips or the hair against ultraviolet rays, to improve the structural quality of the skin or to combat the ageing of the skin.

**12.** Method for beauty treatment consisting in applying to the skin, the lips or the hair a cosmetic composition comprising a compound of formula I as defined in claim 1.

**13.** Use of an Aphloia leaf extract obtained by extraction by a polar solvent or a mixture of polar solvents, or of a Mangifera leaf extract obtained by extraction by a polar solvent or a mixture of polar solvents, to provide a photo-protective effect, an anti-radical action, an anti-elastase action, an anti-collagenase action, an anti-hyaluronidase action or an anti-tyrosinase action.

**14.** Use of purified mangiferin to provide a photo-protective effect, an anti-radical action, an anti-elastase action, an anti-collagenase action or an ant-tyrosinase action.

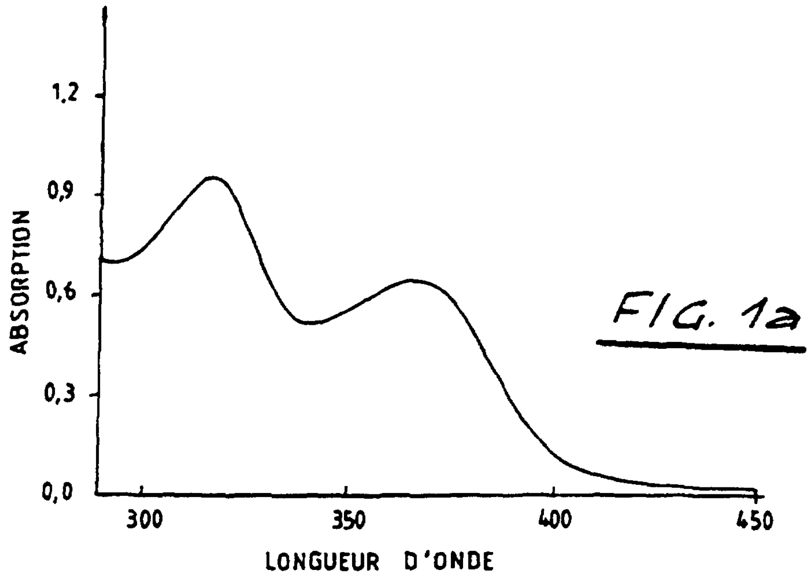


FIG. 1a

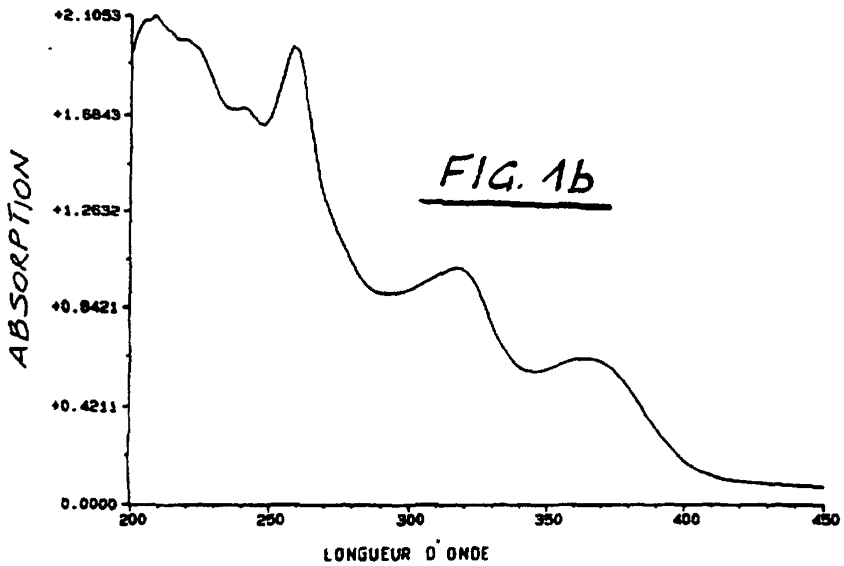


FIG. 1b

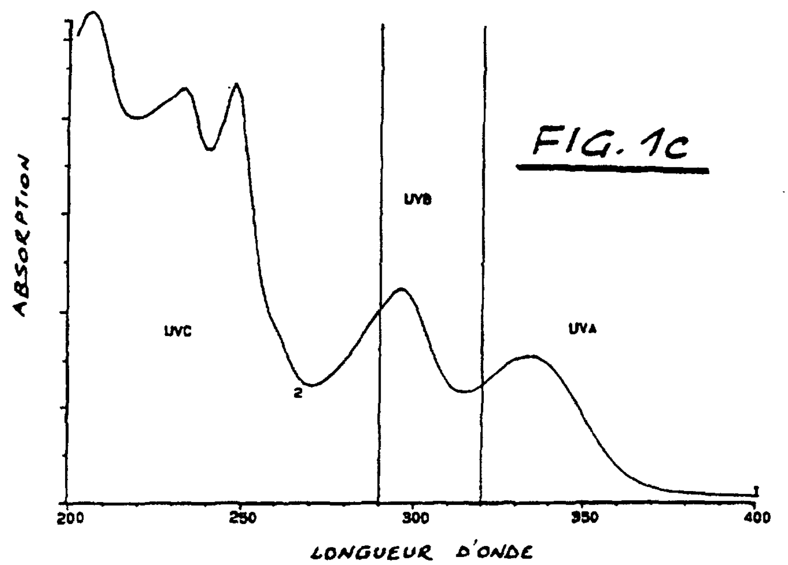


FIG. 1c

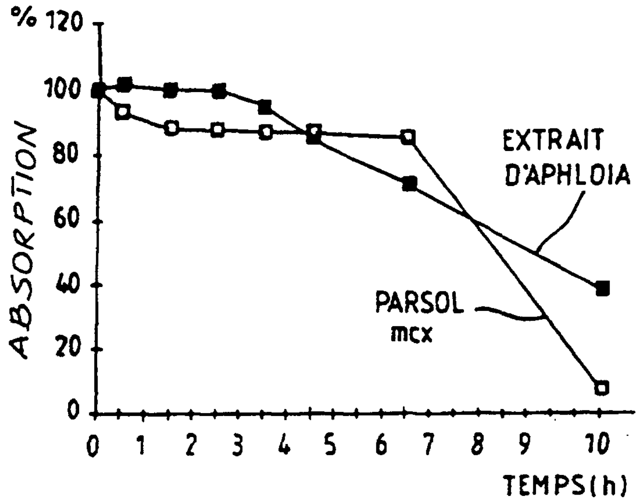


FIG. 2a

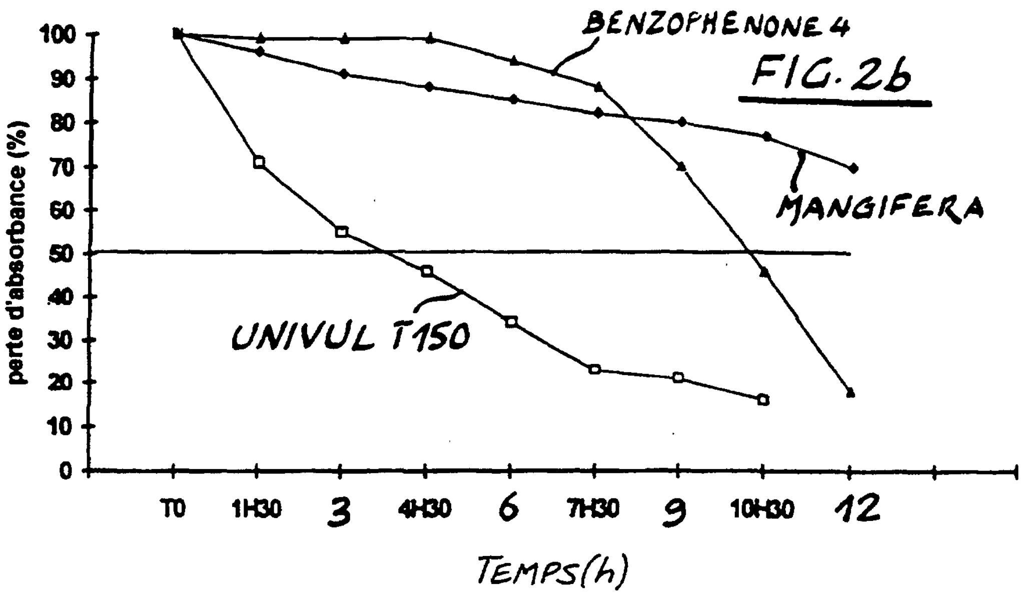


FIG. 2b

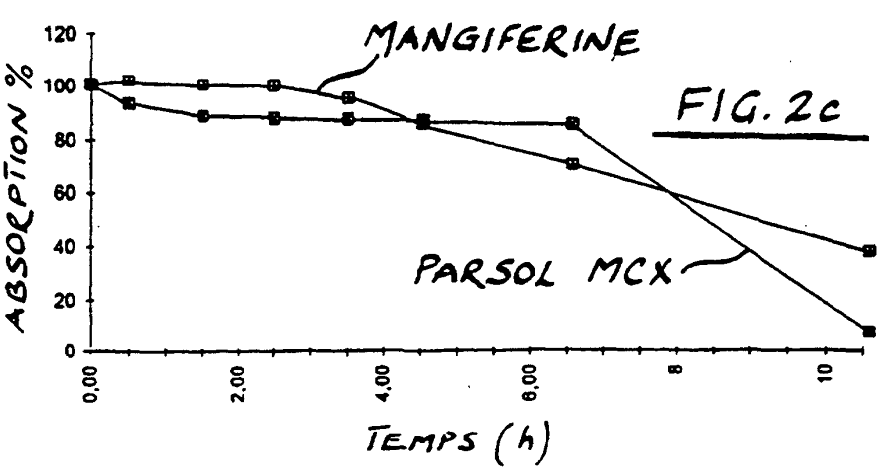


FIG. 2c

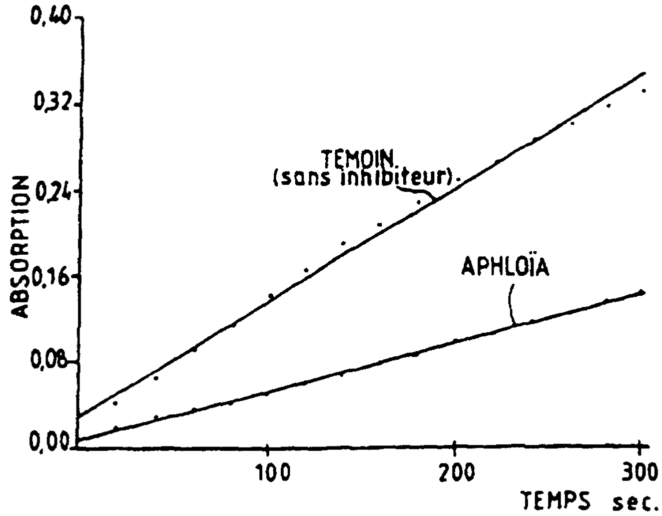


FIG. 3a

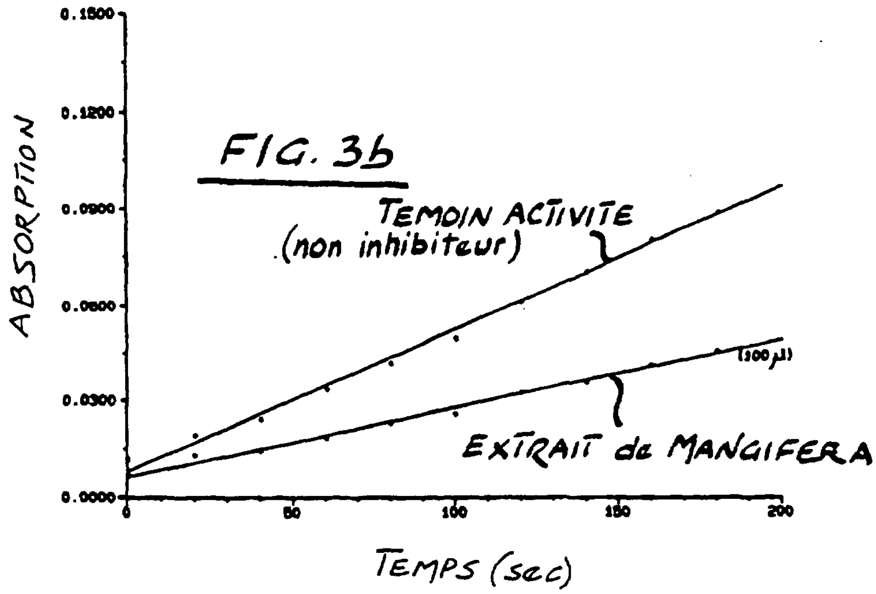


FIG. 3b

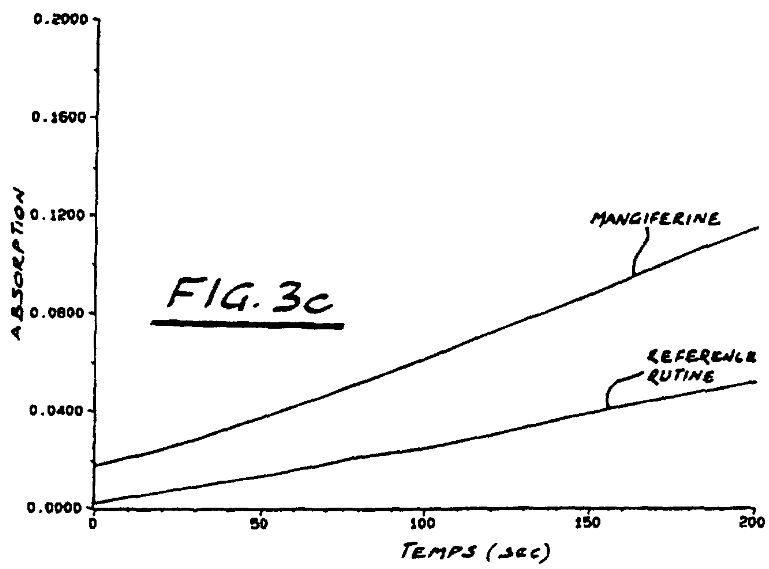


FIG. 3c

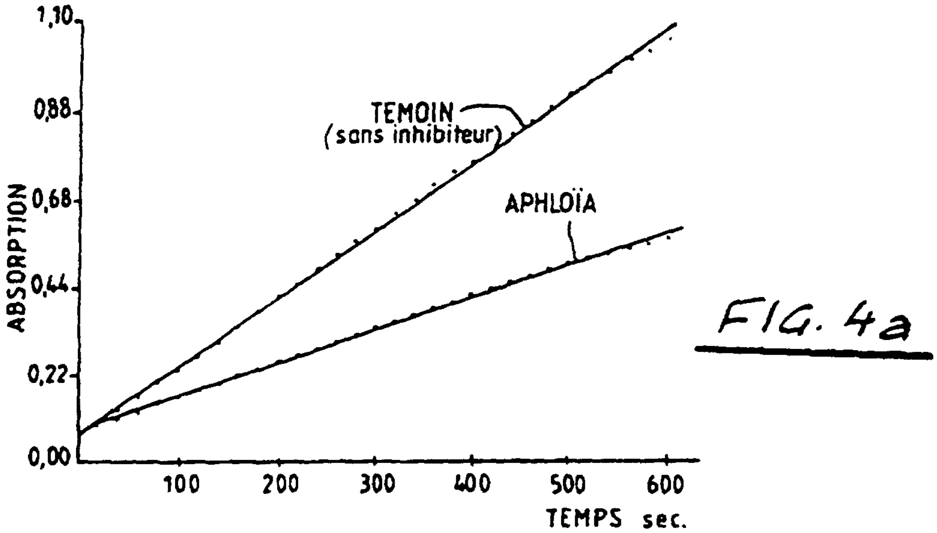


FIG. 4a

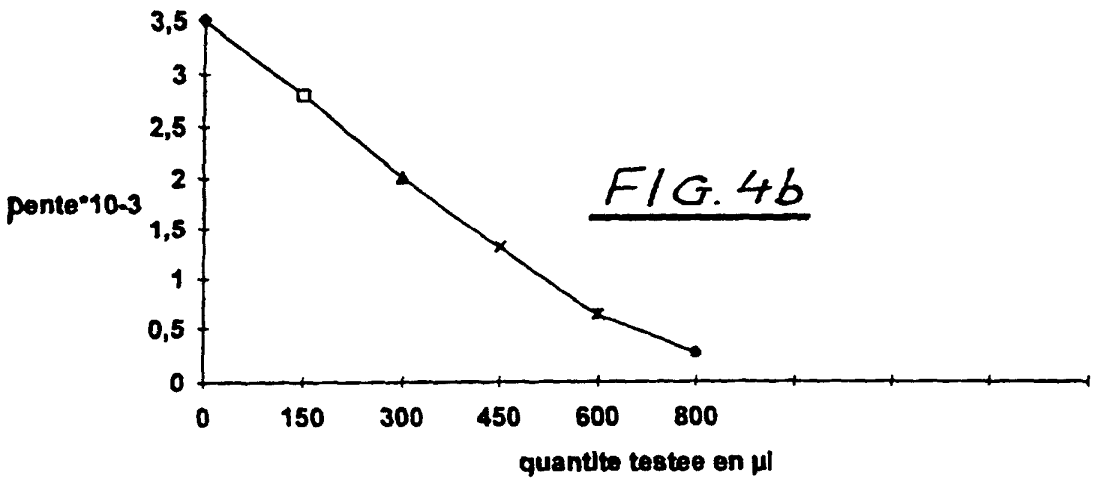


FIG. 4b

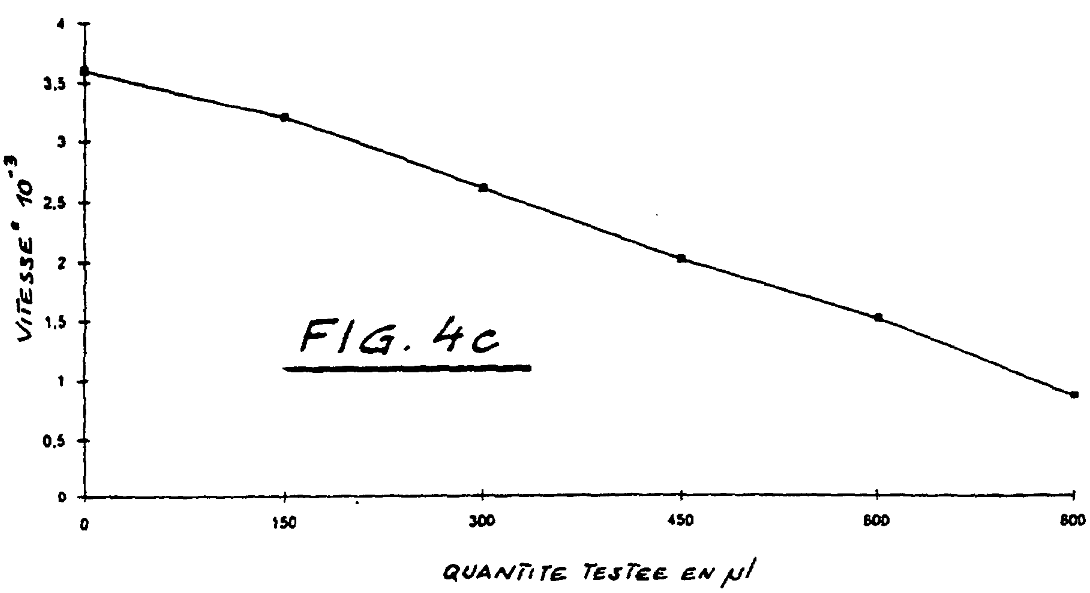


FIG. 4c

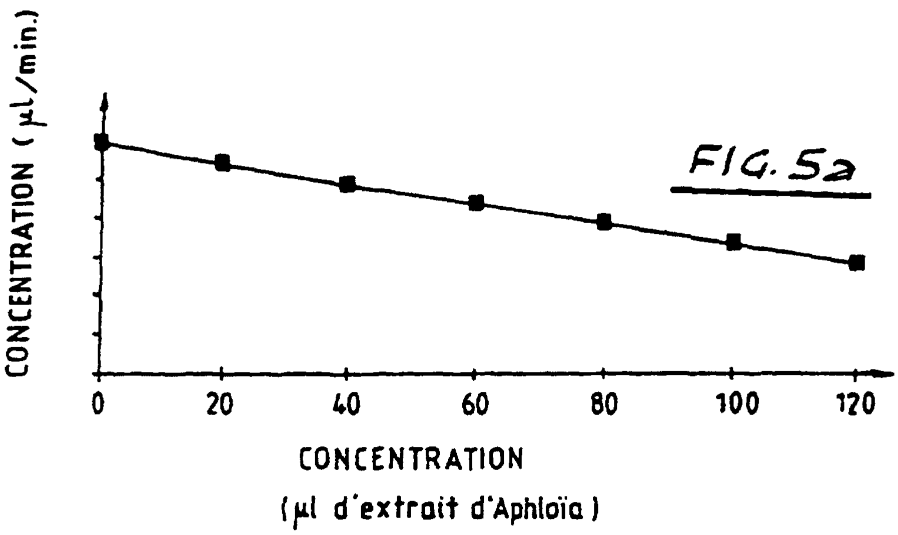


FIG. 5a

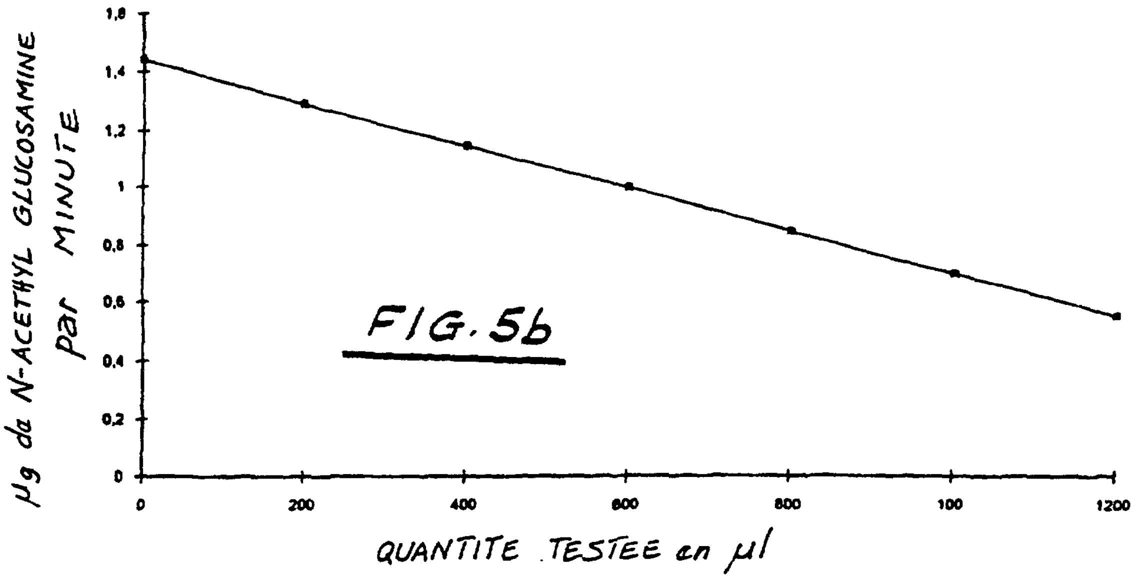


FIG. 5b

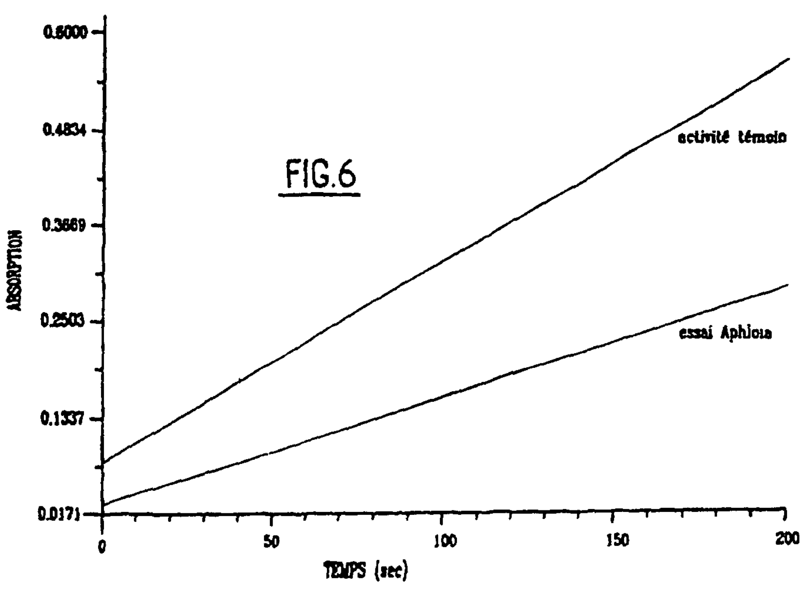


FIG.6

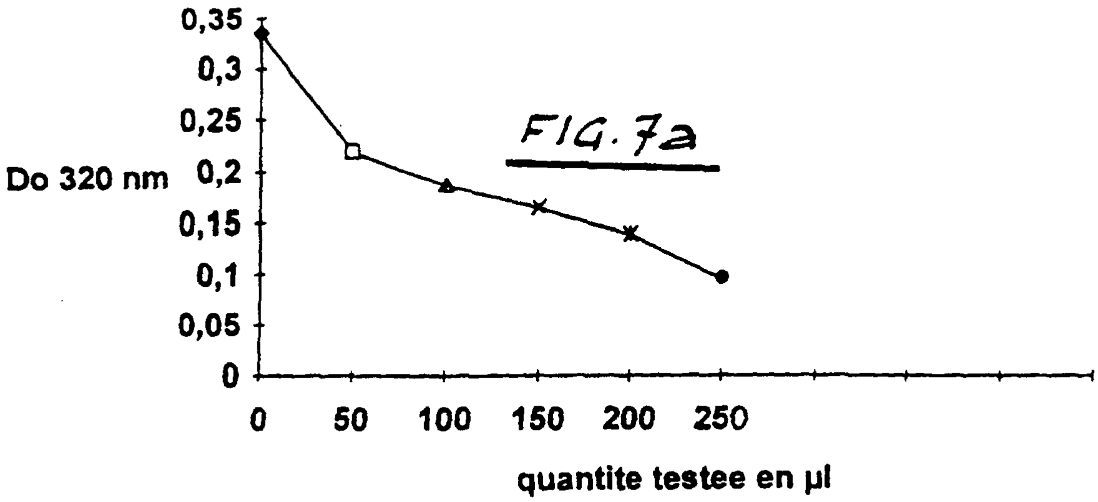
0,35
0,3
0,25
Do 320 nm 0,2
0,15
0,1
0,05
0
0
50
100
150
200
250
quantite testee en µl

FIG. 7a

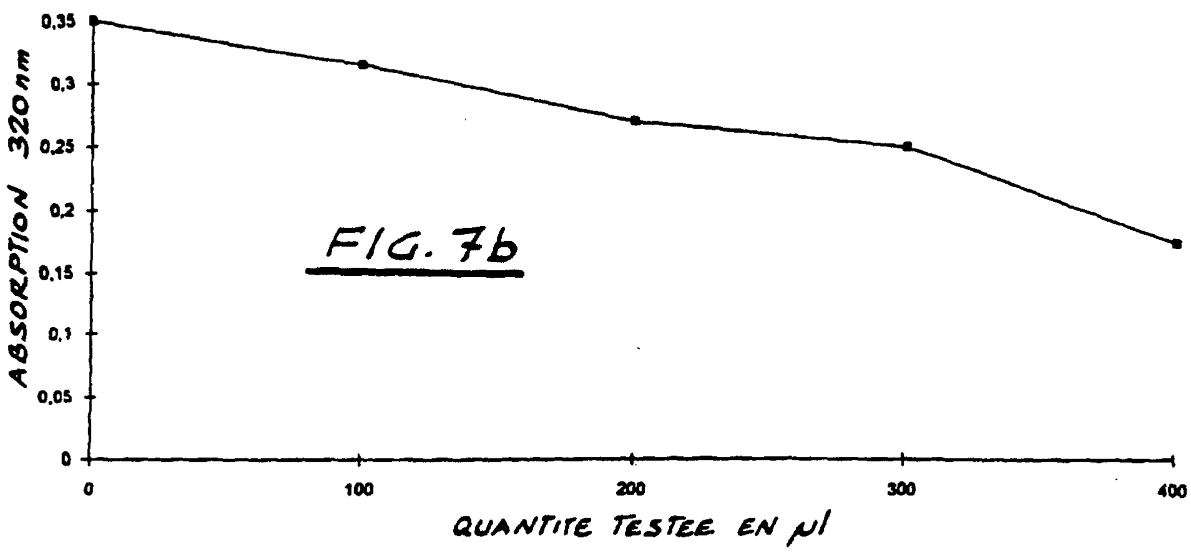
ABSORPTION 320nm
0,35
0,3
0,25
0,2
0,15
0,1
0,05
0
0
100
200
300
400
QUANTITE TESTEE EN µl

FIG. 7b